# EUROPEAN PATENT APPLICATION

(11) **EP 1 721 986 A1**
(43) Date of publication of application: **15.11.2006**
(21) Application number: 06010056.7
(22) Date of filing: 21.10.1998
(51) Int. Cl.: C12N 15/67, C12N 15/63, C12N 5/06

(54) **Rapid generation of stable mammalian cell lines producing high levels of recombinant proteins**

(30) Priority: 29.10.1997 US 63449 P; 20.10.1998 US 175690
(62) Divisional of application: 98953816.0
(71) Applicant: Genetics Institute, LLC, Cambridge, MA 02140 (US)
(72) Inventor: Tomkinson, Kathleen, Cambridge, MA 02138 (US); Davies, Monique, Arlington, MA 02174 (US); McCoy, John, Reading, MA 01867 (US)
(74) Representative: Hermann, Bettina Celia

(57) **Abstract**

The present invention provides a recombinant DNA sequence comprising the DNA sequence of pHTop.

## Description

### FIELD OF THE INVENTION

The present invention relates to a novel method of generating stable mammalian cell lines which produce high levels of recombinant proteins, and to the cell lines and vectors which are suitable for use in such method.

### BACKGROUND OF THE INVENTION

Mammalian cell lines, such as Chinese hamster ovary (CHO) cell lines, are often used for the production of recombinant proteins. In such methods, it is desirable to generate stable cell lines and to be able to generate such high-producing stable cell lines in a relatively short period of time. The generation of such cell lines enables the rapid production of quantities of recombinant protein on a scale useful for purposes of biological evaluation and commercial production.

### SUMMARY OF THE INVENTION

The present invention relates to novel mammalian expression vectors which allow the establishment in a relatively rapid period of time, preferably as short as about 4 weeks, stable mammalian cell lines producing high levels of secreted and membrane-bound recombinant proteins. In a preferred embodiment of the invention, the mammalian cell lines are of Chinese hamster ovary (CHO) origin.

In one embodiment, the present invention comprises recombinant DNA sequences and gene expression plasmids useful for the generation of stable cell lines. Preferred embodiments comprise the recombinant DNA sequences of the gene expression plasmids pHTOP or pHTOP6. In other embodiments, the invention comprises recombinant DNA vectors comprising a gene encoding a chimeric transcription factor [tTA], which tTA may comprise a fusion of an *E. coli* tetracycline repressor protein [*tet* R] to a transcriptional activation domain of herpes simplex virus 16 (VP16); and a vector comprising a minimal promoter preceded by multiple *tet* operator [*tet* O] sequences.

The pHTop vector when transfected into the CHO/A2 cell line leads to very efficient expression of a gene cloned into it, as well as that of DHFR present on the same polycistronic message. Using a stringent MTX selection protocol, high- expressing clones can be isolated and expanded in one month. For five genes tested, expression levels are higher than COS transient expression levels. Expression levels can be amplified by growing cells in increasing concentrations of MTX. It has been demonstrated that stability of expression can be maintained for at least three weeks in the absence of selection.

The level of expression achieved through the one-step selection protocol varies from gene to gene. Secreted protein levels ranging from 1-14 µg/ml have been observed. Stringent MTX selection generally produces clones expressing uniform levels of protein, eliminating the need to screen large numbers of clones. Both secreted and membrane proteins can be expressed at high levels using the pHTop vector.

The streamlined protocol for establishing CHO stable cell lines described in the present invention can be used as an alternative to large-scale COS transfections. CHO cells grow well in serum-free media and conditioned media is easily generated for purification of novel proteins, for example, such protocol may accelerate the generation of stable cell lines in search of a function.

### BRIEF DESCRIPTION OF THE FIGURES

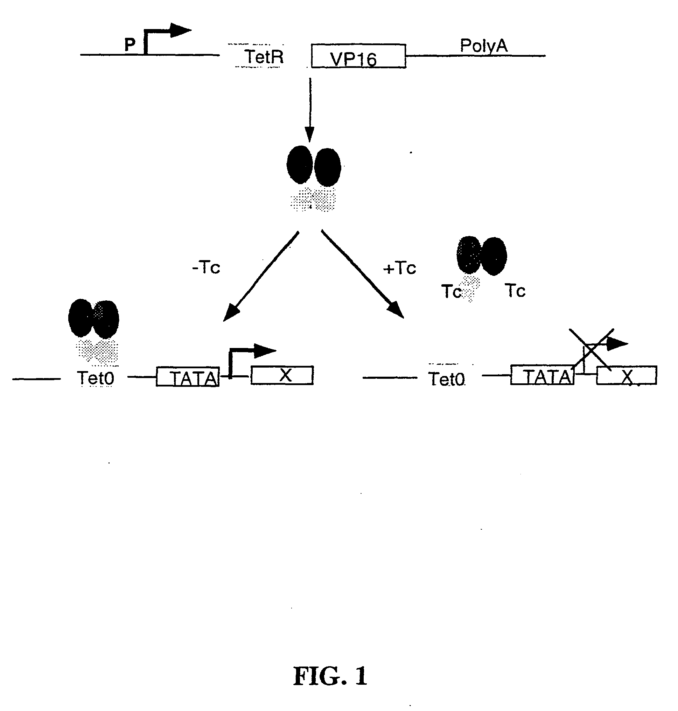

Figure 1 is a diagram of the regulatable expression system characterized by Gossen and Bujard). This expression system is based on two elements: a chimeric transcription factor (tTA), which is a fusion between the *E. Coli* tetracycline repressor (*tet*R) and the transcriptional domain of herpes simplex virus 16 (VP16); and a vector in which a minimal promoter providing a TATA box is preceded by multiple *tet* operator (*tet*O) sequences. When the chimeric transactivator is expressed, its *tet*R domain binds to the *tet*O sequence. This, in turn, brings the strong activation domain VP16 in proximity to the basal transcription complex and activates it. This interaction can be reversed through the action of tetracycline which can therefore be used as a switch to turn transcription "off." In the absence of tetracycline, expression is "on."

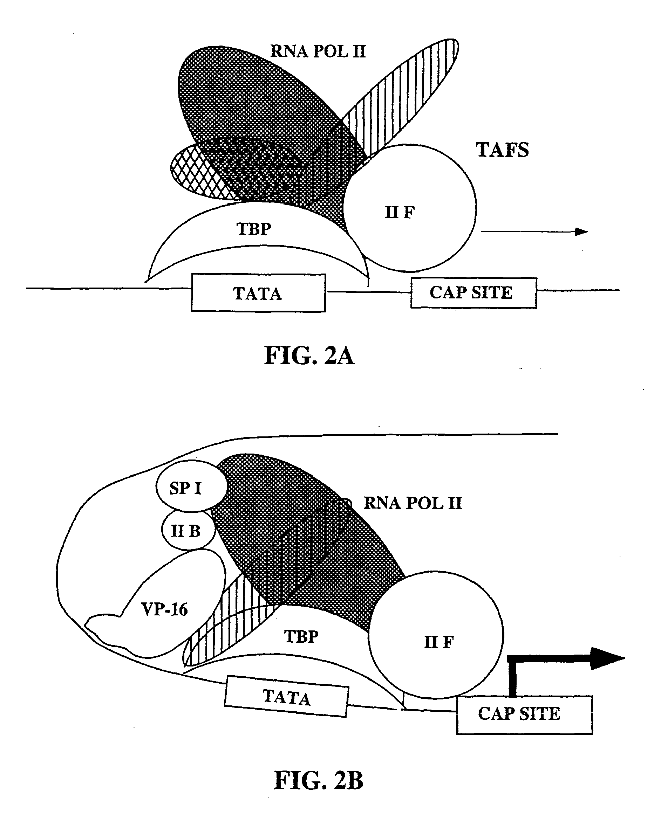

Figure 2a is a diagram showing the basal initiation complex with the minimal promoter in the expression system. Figure 2b shows the activated initiation complex.

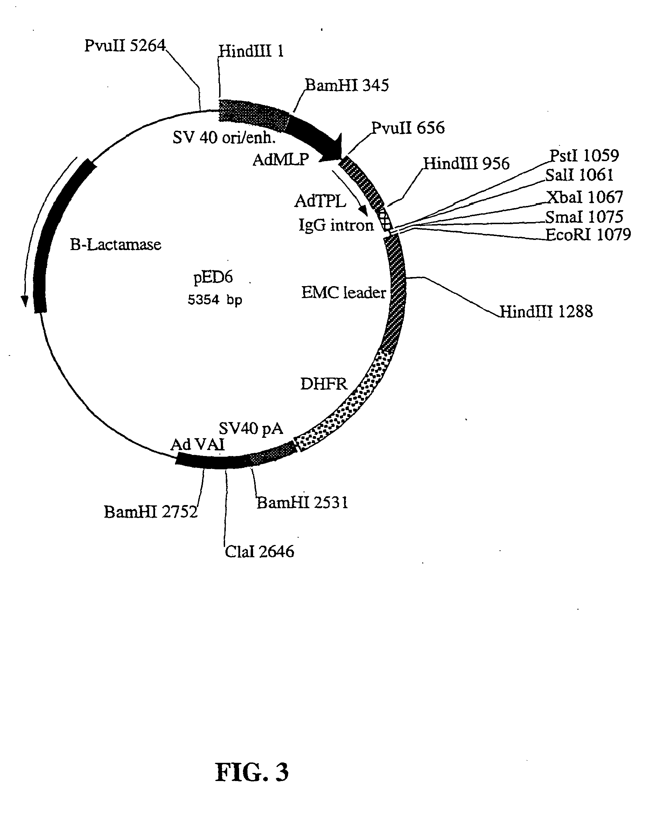

Figure 3 is a plasmid diagram of pED6. PED6 is a plasmid of 5354 base pairs, containing a junction between the EMC leader and DHFR sequences.

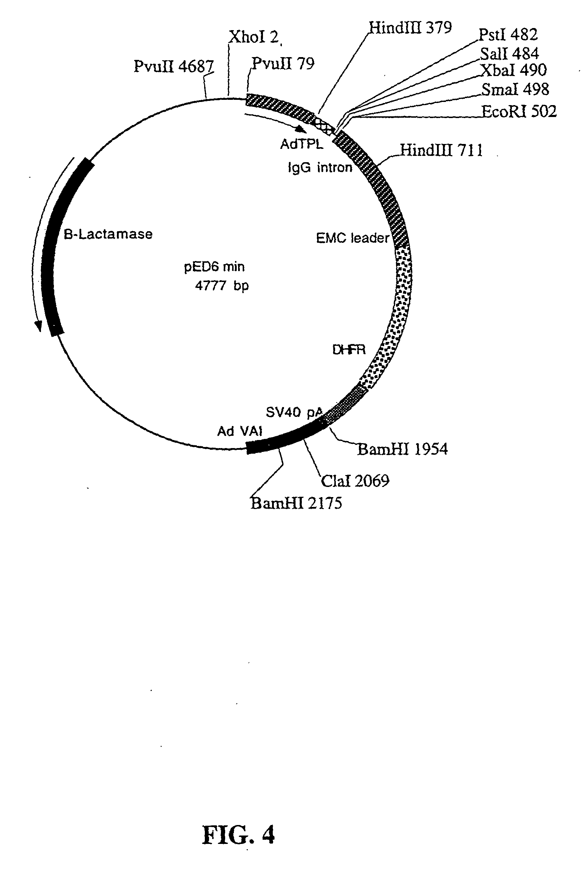

Figure 4 is a plasmid diagram demonstrating the construction of a minimal AdMLP expression plasmid [pED6 min]. The SV40 origin and enhancer, as well as the Adenoviral major late promoter up to 8 bp from the TATA box have ben looped out of pED6, a 575 bp deletion. A unique XhoI site is inserted to allow for the insertion of *tet*O.

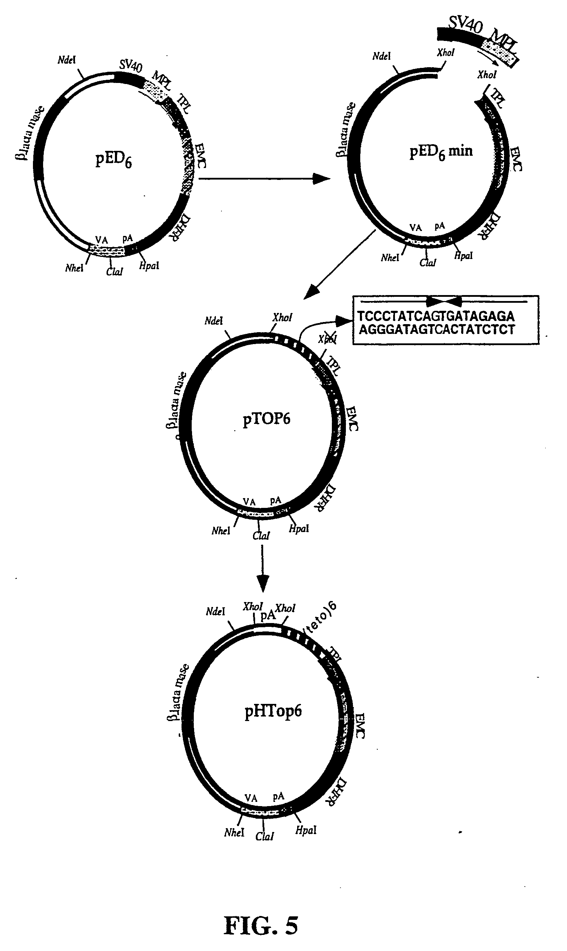

Figure 5 is a diagram showing the construction of pHTop6 from pED6. Following the construction of pED6min, 6 *tet*O sequences are ligated into the XhoI site to form pTOP6. Next the HBV poly A sequences are inserted upstream of the *tet*O sequences.

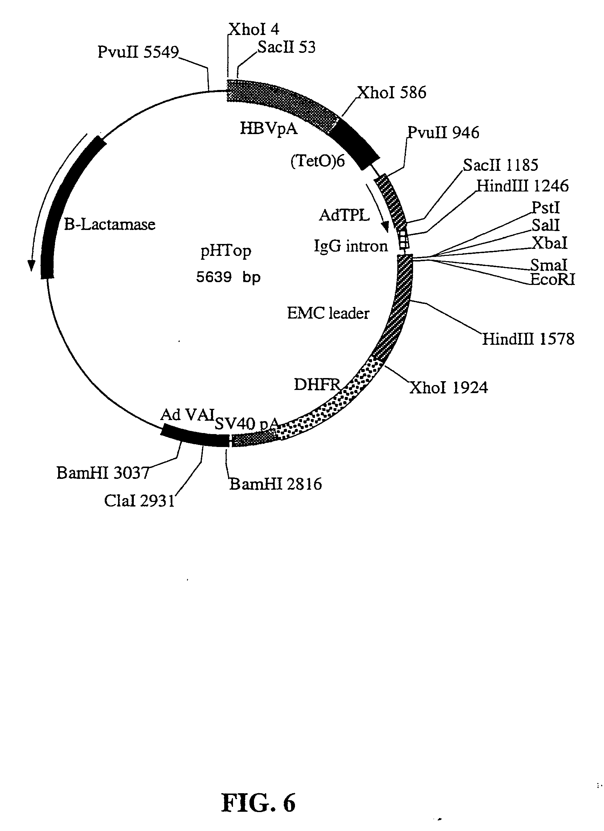

Figure 6 is a plasmid diagram of pHTOP. pHTOP is a 5639 bp plasmid in which the EMC leader/DHFR junction of pHTOP6 is changed to 'cripple' DHFR translation without affecting the level of expression of the upstream gene. This allows the maintenance of stringent selection without the use of high levels of methotrexate [MTX].

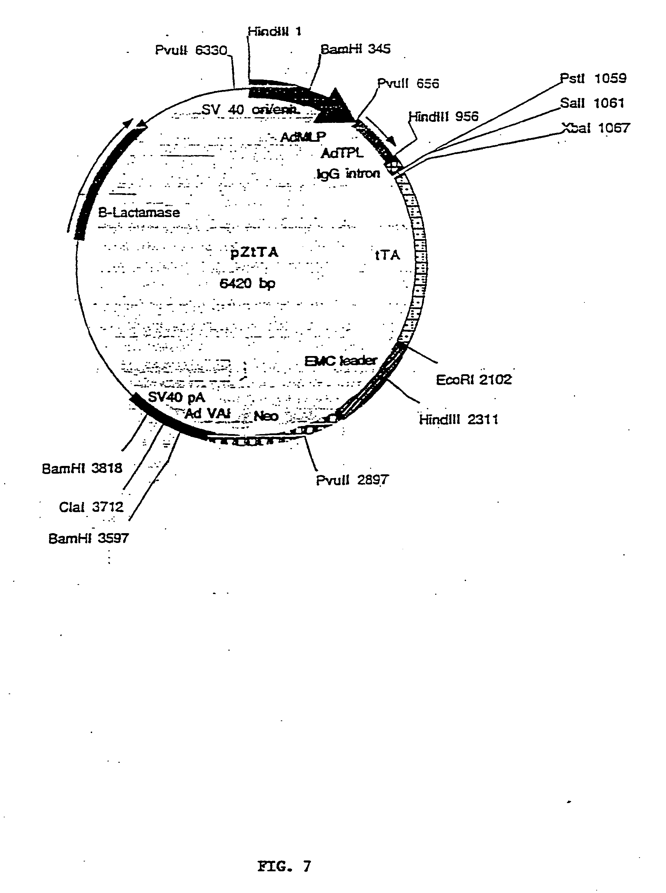

Figure 7 is a plasmid diagram of pZtTA. pZtTA is a 6420 bp plasmid which is used to construct a transactivator CHO cell line. In order to construct such a cell line, pZtTA is electroporated into CHO cells. After electroporation, 24 clones are selected in the presence of 1 ug/ml G418. These 24 clones are then transiently transfected into pTOP6 containing the CAT gene [pTOP6CAT] for selection. The transfected clones are assayed for CAT activity. The leval of activity is compared to that obtained by transfecting pTOP6CAT and pEDtTA into CHO cells.

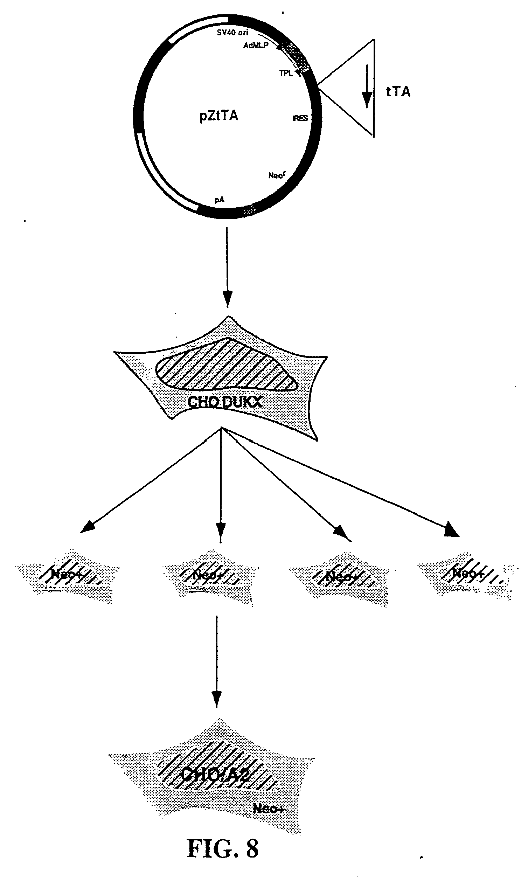

Figure 8 is a diagram showing the derivation of a CHO cell line expressing tTA, CHO/A2, as described in the description of Figure 7 above.

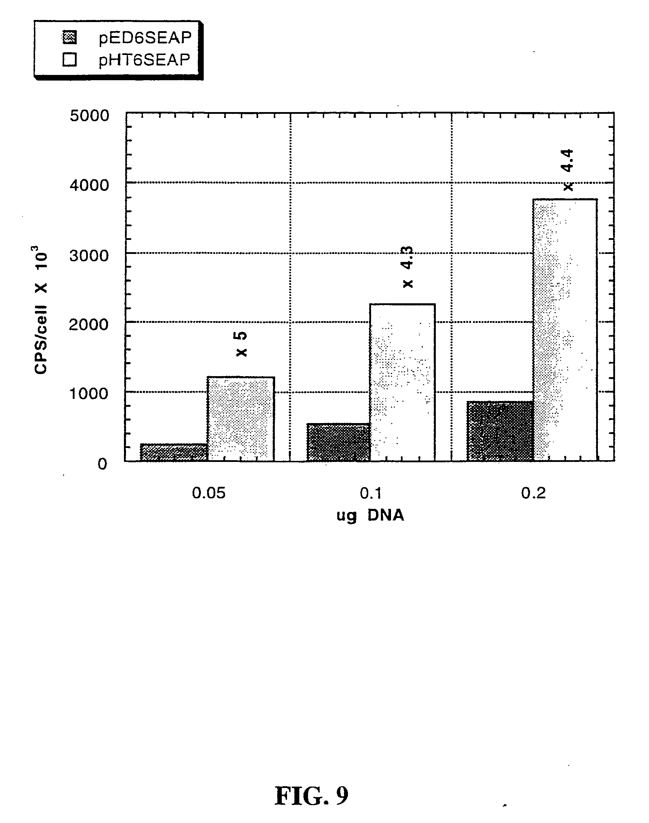

Figure 9 is a graph showing the transient expression of the reporter gene secreted alkaline phosphatase (SEAP) in two cell lines, pEDSEAP and pHTOPSEAP. SEA expression is monitored using a very sensitive chemiluminescent assay. 0.05, 0.1 and 0.2 ug of pEDSEAP and pHTOPSEAP were each lipofected into the CHO/A2 cell line. SEA activity was determined in counts per second [CPS], which are proportional to SEA activity in the range shown on the graph. In the transient transfection experiments shown in Figure 9, the *tet* regulatable promoter is demonstrated to be approximately 5-fold stronger than the adenoviral major late promoter.

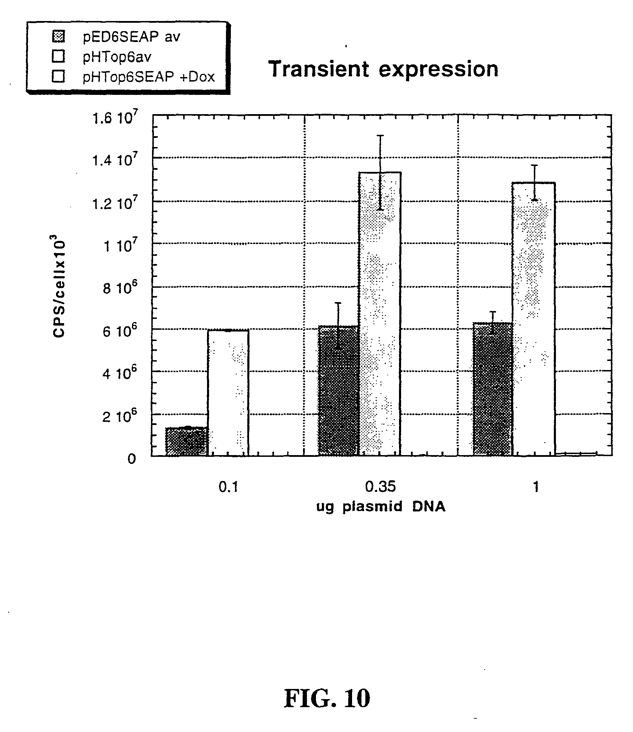

Figure 10 is a graph showing the transient expression of SEA in cells adenovirally transfected with pED6SEAP and pHTOP6SEAP, and pHTOP6SEAP in the presence of Doxycycline, an analog of tetracycline. Again, SEA is shown to be expressed approximately 5-fold more strongly under the regulation of pHTOP6 than under pED6. Addition of Dox completely inhibits DNA expression by the *tet* promoter.

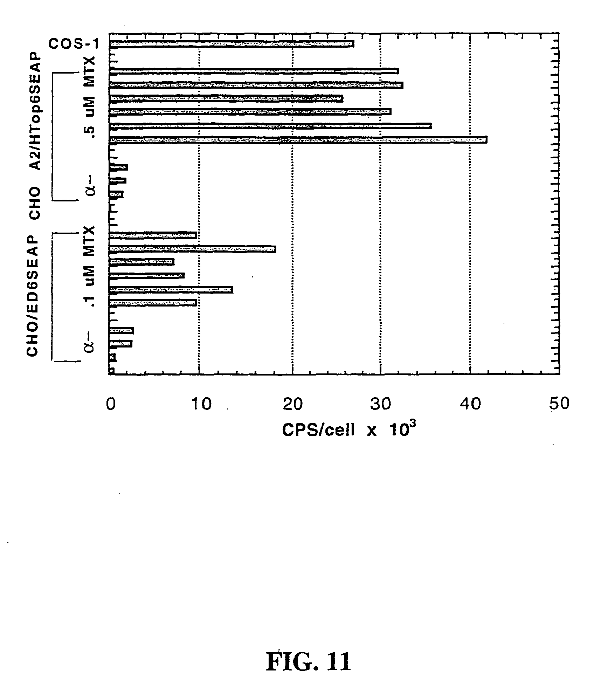

Figure 11 is a graph comparing the level of stable expression of SEA in CHO/A2 cell lines transfected with pED6SEAP and pHTOP6SEAP. Clones were picked in the highest MTX concentrations possible for each vector [0.1 uM MTX for CHO/ED6SEAP; 0.5 uM MTX for CHOA2/HTOP6SEAP]. The expression level observed in the concentration with MTX was about 30 fold higher than that observed in clones picked in alpha [no MTX]. The expression level in pHTOPSEAP is approximately 3-fold higher than that for pED6SEAP. The expression level in these clones is very high for CHO cell lines, as high as that obtained in COS-1 transient transfections.

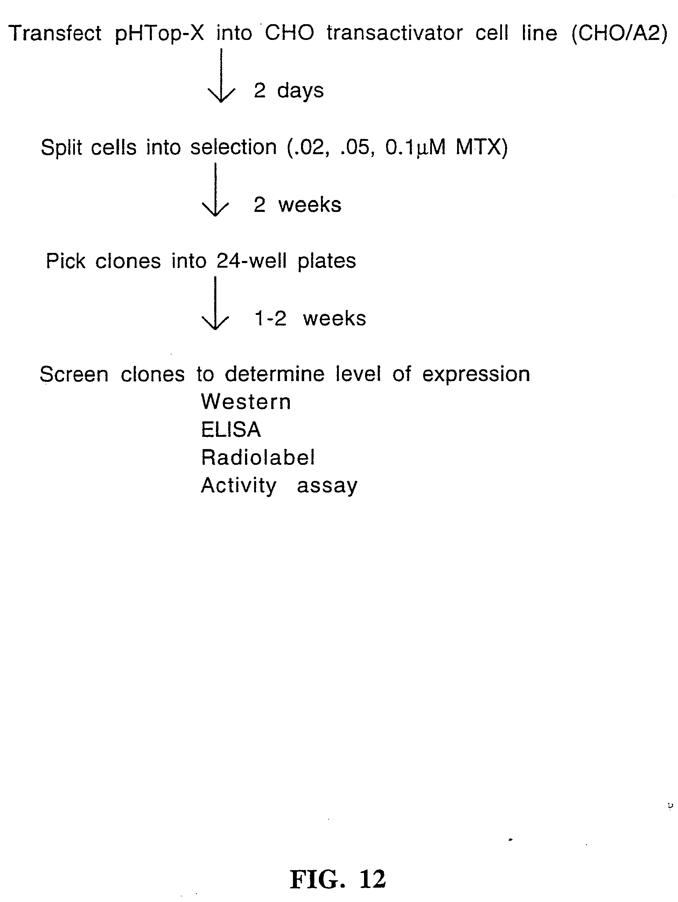

Figure 12 is a diagram of a streamlined protocol for one-step CHO cell line selection using the pHTOP vector and CHO/A2 transactivator cell line.

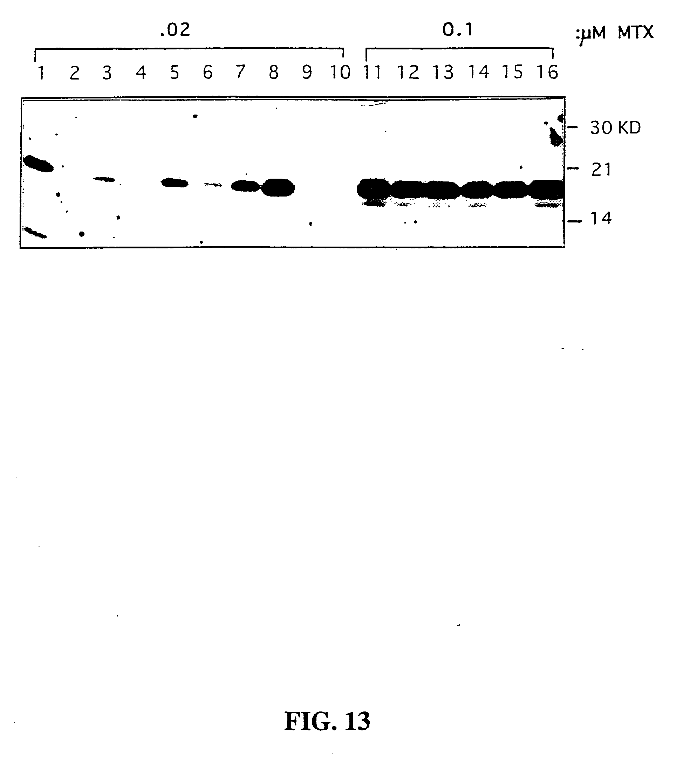

Figure 13 is the Western blot analysis of CHO cells stably expressing hGDF-9, which were established by transfection using the pHTOP vector. 48 hours post-transfection, cells were plated for colony formation in 0.02 and 0.1 uM MTX. After two weeks, clones were picked from each MTX concentration. Cells were grown to confluence and 24 hour serum-free conditioned media was harvested for western analysis using a GDF-9 specific polyclonal antibody followed by chemiluminescent detection.
   Clones selected in 0.1 uM MTX (lanes 11-16) expressed higher levels of GDF-9 compared with clones selected in 0.02 uM MTX (lanes 1-10). Clones selected in the lower MTX concentration displayed a wide range of expression. However GDF-9 expression levels were very uniform for clones selected in 0.1 uM MTX. Thus, stringent selection by increasing the concentration of methotrexate yields clones expressing uniformly high levels of protein.

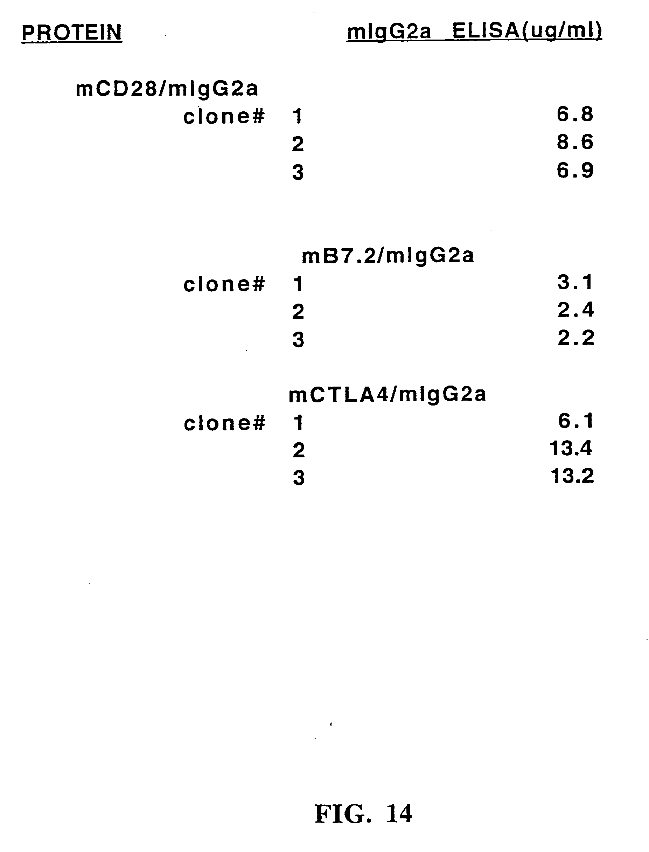

Figure 14 is a graph demonstrating that the level of expression obtained with one-step selection varies from gene to gene. CHO cells which stably express secreted forms of mCD28, mB7.2 or mCTLA4 [all as mIgG2a fusion proteins) were established using the phTOP vector. Clones were selected in 0.05 uM MTX, grown to confluence and 24 hour serum-free conditioned media was harvested for mIgG2a ELISA., For the three genes expressed, mCTLA4 clones produced the highest protein levels [13 ug/ml] followed by mCD28 [8 ug/ml] and mB7.2 [3 ug/ml].

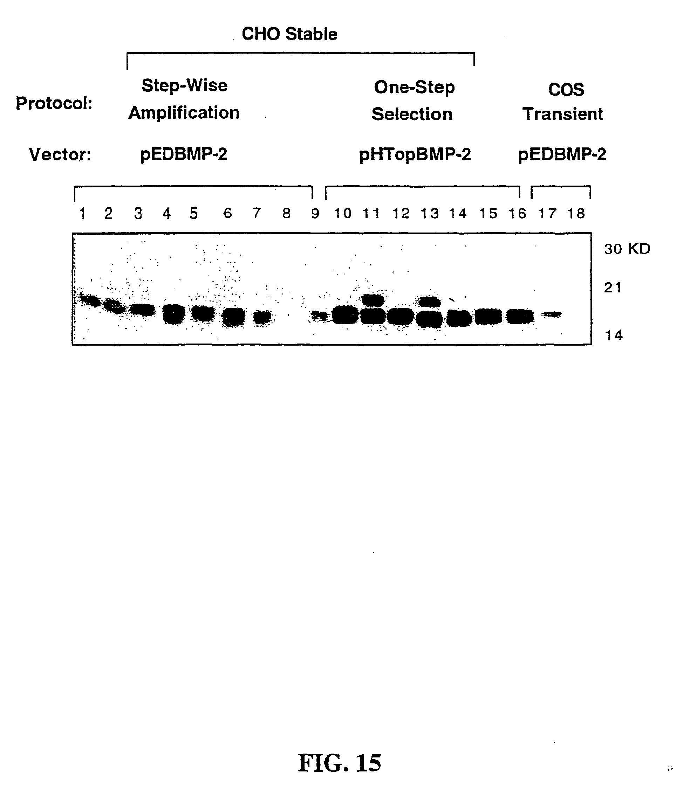

Figure 15 is a Western Blot Analysis showing a comparison of mammalian expression systems. Lanes 1 through 9 show the,effect of step-wise amplification of CHO cells stably transfected with pEDBMP-2. Lanes 10 through 16 show the effect of one-step selection of CHO cells stably stransfected with pHTOPBMP-2. Lanes 17 and 18 show COS transient transfection with pEDBMP-2 and a mock transfection.

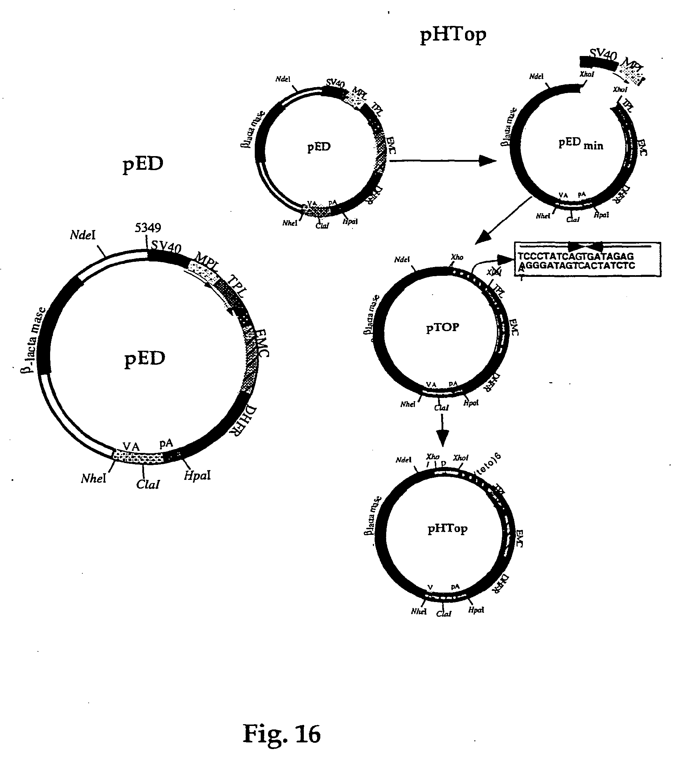

Figure 16 demonstrates the vectors of two efficient expression vectors. pED uses the adenovirus major late promoter [AdMLP] for transcription initiation. It contains the SV40 origin of replication for transient expression in COS cells. A hybrid intron is followed by unique cloning sites for insertion of cDNAs. An internal ribosomal initiation site (EMCV leader) preceding the selectable marker, DHFR, allows that marker to be independently translated from a unique polycistronic message. To make pHTop, the vector pED was modified so that all SV40 and AdMLP promoter enhancer elements were removed and only the TATA box and 35 bp upstream of the transcriptional start site remained [pEDmin]. In its place 6 repeats of the tet operator sequence were inserted. This vector [pTOP] is now uniquely controlled by the transactivator [tTA] through its interaction between the tet repressor and tet operator and tetracycline can be used to switch transcription on and off. In addition, the hepatitis polyadenylation site was inserted upstream of the tet operator sequence to prevent possible interference from cryptic promoters within the plasmid backbone.

### DETAILED DESCRIPTION OF THE INVENTION

The vectors of the present invention make use of strong transcriptional elements activated by a chimeric transactivator construct stably expressed in a recipient mammalian cell line. In a preferred embodiment, the strong transcriptional elements comprise multiple copies of the tet operator positioned upstream and adjacent to a minimal mammalian "TATA" sequence. This chimera forms a strong mammalian promoter. In a further preferred embodiment, this strong promoter directs the synthesis of a polycistronic message, in which expression of a resistance marker, such as the dihydrofolate resistance gene [DHFR], is linked to the expression of the gene of interest, allowing selection of highly expressing clones in a one-step selection process. In a preferred embodiment, a portion of a suitable leader sequence, such as the EMC virus [EMCV] leader, may be used for the efficient functioning of this polycistronic message. The EMCV leader sequence may be obtained, for example, from pMT2-ECAT1 [S.K, Jung, et al, J. Virol 63:1651-1660 (1989)]. The disclosure of this document is hereby incorporated herein by reference. The combined use of this strong chimera promoter, which produces a polycistronic message with a selectable resistance marker, with a host cell which expresses a chimeric transactivator, allows the rapid selection of cell lines producing high levels of recombinant protein in one step.

Secreted protein levels of up to approximately 50 ug/ml have been obtained using this one-step selection method. Clones stringently selected in high levels of methotrexate [MTX] produce uniform levels of protein, eliminating the need to screen large numbers of clones. Although the production level obtained with the initial selection step is variable from gene to gene, levels can be amplified by increasing the concentration of MTX. In addition, production levels are stable in the presence of selection or for at least 3 weeks when selection pressure is removed.

The present system has the advantage of developing stable cell lines much more rapidly than was previously possible using the usual step-wise amplification [approximately 1 month compared to approximately 4 months]. In addition, higher production levels can be obtained with this system compared to transient expression in COS cells. Accordingly, the present invention provides methods for developing high-expressing stable cell lines quickly and easily, filling the long-felt need for such systems between transient expression in COS cells [fast but labor intensive for large scale production of proteins] and stable expression by step-wise amplification [slow and labor intensive].

The expression system is based on the combined use of two elements: a chimeric transcription factor [tTA], which is a fusion between the *E*. *coli* tetracycline repressor [*tet* R] and the transcriptional domain of herpes simplex virus 16 (VP16), and a vector in which a minimal promoter providing a TATA box is preceded by multiple *tet* operator [*tet* O] sequences. These operators bring the strong activation domain of VP16 in close proximity to the basal transcription complex, activating it. This interaction can be reversed through the use of tetracycline, which can therefore be used as a switch to turn transcription "off." In the absence of tetracycline, expression is "on." However, the present method does not require that this regulation be used. Thus, in one embodiment, the present invention comprises a plasmid in which a minimal promoter is operably linked to a leader sequence which in turn is operably linked to a DHFR gene. Upstream of the leader sequence are one or more restriction sites suitable for the insertion of a gene encoding a desired protein for expression. In a preferred embodiment, the plasmid pHTOP6 is used, which is created from the plasmid pED as described further herein. The plasmid pHTOP is also useful in the present invention. In pHTOP, the junction between the EMCV leader and DHFR gene was altered to impair DHFR translation without affecting the level of expression of the upstream gene. For the production of larger proteins, where low levels of DHFR expression may be expected, pHTOP6 may be the preferred vector. The methods of the present invention are useful for the production of both secreted and membrane proteins. The methods of this invention can be used as an alternative to large-scale COS transfections. In the preferred embodiment wherein CHO cells are used, the cells can be grown in serum-free media for the purification of proteins.

A transactivator cell line useful as a recipient mammalian cell line may be derived by transfecting a chimeric transcription factor, such as tTA described above, into suitable cell lines, such as a CHO cell line. In a preferred embodiment, the CHO cell line CHO DUKX B11, which is deficient in DHFR and therefore not normally able to survive in the presence of methotrexate selection, is used. A diagram of such a process is shown in Figures 10 and 11.

The plasmid pHTOP-X, where X is the coding sequence for the protein to be expressed is made and transfected into the transactivator cell line, and screened as illustrated in Figure 15. Thus, in one embodiment, the present invention comprises recombinant DNA sequences comprising the DNA sequence of pHTOP or pHTOP6. In other embodiments, the present invention comprises methods for producing a stable recombinant mammalian cell line, said method comprising: (1) transfecting a recipient mammalian cell line with a plasmid vector to form a transfected recipient mammalian cell line, said plasmid vector comprising: (a) a minimal promoter preceded by multiple tet operators; (b) a leader sequence capable of directing the efficient expression of a polycistronic message; and (c) a polycistronic message comprising a first DNA encoding a protein of interest and a second DNA sequence encoding a selectable marker gene, and said recipient mammalian cell line comprising (1) a chimeric transcription factor, which comprises a fusion of one or more copies of an *E*. *Coli* tetracycline repressor; (2) a transcriptional domain of herpes simplex virus 16.; and (3) a vector comprising a minimal promoter preceded by multiple *tet* operator sequences; and (B) isolating the resulting said transfected recipient mammalian cell line. The transfected cell line may optionally be cultured for further use.

The examples below describe some of the preferred embodiments of the present invention.

A. In step-wise amplification, the following was observed CHO DUKX cells were stably transfected with pEDBMP-2. Cells were plated for colony formation in alpha media 48h post-transfection. After 2 weeks, clones were picked, grown in .02:M MTX until stable (4 weeks) then transferred to 0.1:M MTX and grown for 4 weeks. Serum-free conditioned media (24h) was harvested from confluent cells for western analysis.

B. In the present one-step selection CHO DUKX/A2 cells were stably transfected with pHTopMBP-2. 48h post-transfection, cells were plated for colony formation in 0.1M MTX and 1mg/ml G418 media. Two weeks later, clones were picked, grown to confluence and serum-free conditioned media was harvested from confluent cells for western analysis.

C. COS transient transfections were accomplished using COS-1 cells transiently transfected with pEDBMP-2. 48-72h post-transfection, serum-free conditioned media was harvested for western analysis.

### Western analysis:

10:1 conditioned media from protocols A, B, C above were run on a 16% SDS-PAGE gel under reducing conditions and tranferred to nitrocellulose by western blot. BMP-2 was detected with a BMP-2 specific polyclonal antibody followed chemiluminescent detection.

Clones established by step-wise amplification using the pED vector displayed a wide range of BMP-2 expression. In contrast, clones picked in the one-step selection using the pHTop vector showed higher and more uniform BMP-2 expression. The level of BMP-2 expressed transiently in COS cells lane was much lower than that seen in the pHTop clones.

Stable CHO cell lines established using one-step selection displayed consistently higher expression levels compared with CHO step-wise amplification or COS transient. In addition, expression levels achieved with one-step selection wee more uniform. CHO cell lines stably expressing a secreted form of a mB7.1-mlgG2a fusion protein were established by transfection using the pHTop vector. Clones selected in 0.1:M MTX were passaged twice a week in the presence or absence of selection (MTX and G418) for 3 weeks. Serum-free conditioned median (24h) was harvested from confluent cells at 1, 2 and 3 weeks and analyzed by western blot using an anti-mlgG2a HRP antibody followed by chemiluminescent detection. Expression levels remained constant for at least 3 weeks when selection is removed from cells selected in high concentrations of methotrexate.

CHO cells stably expressing a secreted from of a hCD28-hlgG4 fusion protein were established by transfection using the pHTop vector. Clones were selected in 0.1:M MTX, then grown in 0.5:M MTX for 3 weeks. Serum-free conditioned media (24h) harvested from confluent cells was analyzed by western blot using anti-hlgG-HRP antibody followed by chemiluminescent detection. Each lane represents 101 conditioned media or purified hlgG4 protein for quantitation. Expression levels for conditioned media from the three highest expressing clones (2, 3, 5) were measured by hlgG4 ELISA.

Clones selected in 0.1:M MTX express approximately 1-2:g/ml hCD28/hlgg4 showed significant increase (5-10 fold) in hCD28 expression while 2 clones (#1,40 showed a moderate increase in expression. Expression levels of cells selected in high concentrations of methotrexate can be amplified by increasing the concentration of methotraxate.

CHO cell lines were established by transfecting pHTopmuFrzb-1 (murine Frazzled) and selecting clones in 0.05 :M MTX. 2 pools of colonies that survived 0.1 µM MTX were also established. Cells were labeled for 6 hr with 35S Methionine/Cysteine and conditioned media was harvested and analyzed by SDS-PAGE. Each lane represents 50 µl of conditioned media from clones and pools. Expression of individual clones selected in 0.05 :M MTX was uniform and the expression from the 2 pools was as high as that of the individual clones.

Due to the uniformity of expression of the individual clones under stringent selection conditions, it is possible to pool colonies without compromising expression, therefore speeding up the last step in the generation of stable cell lines.

CHO cell lines stably expressing CCR5 were established by transfection using the pHTop vector. Clones selected in .02:M MTX were analyzed for CCR5 expression by FACS analysis. Transfected cells were stained using an anti-CCR5 monoclonal antibody (clone 45531.111 from R&D) or a murine lgG2a isotype for untransfected control cells followed by a PE-conjugated anti-murine lgG antibody. Of the 13 clones screened for CCR5 expression, 11 clones expressed CCR5 an demonstrated by a 2 log increase of fluorescence over untransfected control cells. Only 2 clones showed no CCR5 expression above background.

The one step selection system can also be used to express transmembrane proteins.

The examples and figures on the following pages illustrate practice of the present invention in generating stable mammalian cell lines which produce high levels of recombinant proteins, using cell lines and vectors which constitute part of the invention, which are suitable for use in such method. In the examples, it is demonstrated that the present invention is effective for the efficient production of recombinant proteins. A large number of modifications and variations will be apparent to the skilled artisan from reading this specification and the examples. Such modifications and variations constitute part of the invention, and the examples are not limiting.

## Claims

1. A recombinant DNA sequence comprising the DNA sequence of pHTOP.

2. A recombinant DNA vector comprising:
(a) a chimeric transcription factor, which chimeric transcription factor comprises a fusion of an *E. coli* tetracycline repressor with the transcriptional activator domain of herpes simplex virus 16; and
(b) a vector comprising a minimal promoter preceded by multiple *tet* operator sequences.

3. A method for producing a stable recombinant mammalian cell line, said method comprising:
(A) transfecting a recipient mammalian cell line with a plasmid vector to form a transfected recipient mammalian cell line,
(1) said plasmid vector comprising:
(a) a minimal promoter preceded by multiple tet operators;
(b) a leader sequence capable of directing the efficient expression of a polycistronic message; and
(c) a polycistronic message comprising a first DNA encoding a protein of interest and a second DNA sequence encoding a selectable marker gene;
(2) said recipient mammalian cell line comprising a chimeric transcription factor which comprises a fusion of an *E*. *coli* tetracycline repressor and a transcriptional activator domain of herpes simplex virus 16; and
(B) isolating and optionally culturing said transfected recipient mammalian cell line.


SEQUENCE LISTING

(1) GENERAL INFORMATION:

(i) APPLICANT:GENETICS INSTITUTE, INC.

(ii) TITLE OF INVENTION: RAPID GENERATION OF STABLE MAMMALIAN
CELL LINES PRODUCING HIGH LEVELS OF RECOMBINANT PROTEINS

(iii) NUMBER OF SEQUENCES: 1

(iv) CORRESPONDENCE ADDRESS:
(A) ADDRESSEE: GENETICS INSTITUTE, INC.
(B) STREET: 87 CAMBRIDGEPARK DRIVE
(C) CITY: CAMBRIDGE,
(D) STATE: MASSACHUSETTS
(E) COUNTRY: US
(F) ZIP: 02140

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: US XXXX
(B) FILING DATE:
(C) CLASSIFICATION:

(viii) ATTORNEY/AGENT INFORMATION:
(A) NAME: LAZAR, STEVEN R.
(B) REGISTRATION NUMBER: 32,618
(C) REFERENCE/DOCKET NUMBER: GI5310A-PCT

(ix) TELECOMMUNICATION INFORMATION:
(A) TELEPHONE: (617) 498-8260
(B) TELEFAX: (617) 876-5851

(2) INFORMATION FOR SEQ ID NO:1:

(i) SEQUENCE CHARACTERISTICS:
(A) LENGTH: 5639 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

AAGCTCGAGC GCGGGACGTC CTTTGTTTAC GTCCCGTCGG CGCTGAATCCCGCGGACGAC 60
CCCTCTCGGG GCCGCTTGGG AGTCTCTCGT CCCCTTCTCC GTCTGCCGTTCCAGCCGACC 120
ACGGGGCGCA CCTCTCTTTA CGCGGTCTCC CCGTCTGTGC CTTCTCATCTGCCGGTCCGT 180
GTGCACTTCG CTTCACCTCT GCACGTTGCA TGGAGACCAC CGTGAACGCCCATCAGATCC 240
TGCCCAAGGT CTTACATAAG AGGACTCTTG GACTCTCAGC AATGTCAACGACCGACCTTG 300
AGGCCTACTT CAAAGACTGT GTGTTTAAGG ACTGGGAGGA GCTGGGGAGGAGATTAGGT 360
TAAAGGTCTT TGTATTAGGA GGCTGTAGGC ATAAATTGGT CTGCGCACCAGCACCATGCA 420
ACTTTTTCAC CTCTGCCTAA TCATCTCTTG TACATGTCCC ACTGTTCAAGCCTCCAAGCT 480
GTGCCTTGGG TGGCTTTGGG GCATGGACAT TGACCCTTAT AAAGAATTTGGAGCTACTGT 540
GGAGTTACTC TCGTTTTTGC CTTCTGACTT CTTTCCTTCC GTCAGCTCGAGTTTACCACT 600
CCCTATCAGT GATAGAGAAA AGTGAAAGTC GAGTTTACCA CTCCCTATCAGTGATAGAGA 660
AAAGTGAAAG TCGAGGTCGA GTTTACCACT CCCTATCAGT GATAGAGAAAAGTGAAAGTC 720
GAGGTCGAGT TTACCACTCC CTATCAGTGA TAGAGAAAAG TGAAAGTCGAGTTTACCACT 780
CCCTATCAGT GATAGAGAAA AGTGAAAGTC GAGGTCGAGT TTACCACTCCCTATCAGTGA 840
TAGAAAAGTG AAAGTGAAAG TCGAGGTCGA GTCGAGGGGG GCTATAAAAGGGGGTGGGGG 900
CGCGTTCGTC CTCACTCTCT TCCGCATCGC TGTCTGCGAG GGCCAGCTGTTGGGCTCGCG 960
GTTGAGGACA AACTCTTCGC GGTCTTTCCA GTACTCTTGG ATCGGAAACCCGTCGGCCTC 1020
CGAACGGTAC TCCGCCACCG AGGGACCTGA GCGAGTCCGC ATCGACCGGATCGGAAAACC 1080
TCTCGACTGT TGGGGTGAGT ACTCCCTCTC AAAAGCGGGC ATGACTTCTGCGCTAAGATT 1140
GTCAGTTTCC AAAAACGAGG AGGATTTGAT ATTCACCTGG CCCGCGGTGATGCCTTTGAG 1200
GGTGGCCGCG TCCATCTGGT CAGAAAAGAC AATCTTTTTG TTGTCAAGCTTGAGGTGTGG 1260
CAGGCTTGAG ATCTGGCCAT ACACTTGAGT GACAATGACA TCCACTTTGCCTTTCTCTCC 1320
ACAGGTGTCC ACTCCCAGGT CCAACTGCAG GTCGACTCTA GACCCGGGGAATTCTAACGT 1380
TACTGGCCGA AGCCGCTTGG AATAAGGCCG GTGTGCGTTT GTCTATATGTTATTTTCCAC 1440
CATATTGCCG TCTTTTGGCA ATGTGAGGGC CCGGAAACCT GGCCCTGTCTTCTTGACGAG 1500
CATTCCTAGG GGTCTTTCCC CTCTCGCCAA AGGAATGCAA GGTCTGTTGAATGTCGTGAA 1560
GGAAGCAGTT CCTCTGGAAG CTTCTTGAAG ACAAACAACG TCTGTAGCGACCCTTTGCAG 1620
GCAGCGGAAC CCCCCACCTG GCGACAGGTG CCTCTGCGGC CAAAAGCCACGTGTATAAGA 1680

TACACCTGCA AAGGCGGCAC AACCCCAGTG CCACGTTGTG AGTTGGATAGTTGTGGAAAG 1740

AGTCAAATGG CTCTCCTCAA GCGTATTCAA CAAGGGGCTG AAGGATGCCCAGAAGGTACC 1800

CCATTGTATG GGATCTGATC TGGGGCCTCG GTGCACATGC TTTACATGTGTTTAGTCGAG 1860

GTTAAAAAAC GTCTAGGCCC CCCGAACCAC GGGGACGTGG TTTTCCTTTGAAAAACACGA 1920

TTGCTCGAGC CATCATGGTT CGACCATTGA ACTGCATCGT CGCCGTGTCCCAAAATATGG 1980

GGATTGGCAA GAACGGAGAC CTACCCTGGC CTCCGCTCAG GAACGAGTTCAAGTACTTCC 2040

AAAGAATGAC CACAACCTCT TCAGTGGAAG GTAAACAGAA TCTGGTGATTATGGGTAGGA 2100

AAACCTGGTT CTCCATTCCT GAGAAGAATC GACCTTTAAA GGACAGAATTAATATAGTTC 2160

TCAGTAGAGA ACTCAAAGAA CCACCACGAG GAGCTCATTT TCTTGCCAAAAGTTTGGATG 2220

ATGCCTTAAG ACTTATTGAA CAACCGGAAT TGGCAAGTAA AGTAGACATGGTTTGGATAG 2280

TCGGAGGCAG TTCTGTTTAC CAGGAAGCCA TGAATCAACC AGGCCACCTCAGACTCTTTG 2340

TGACAAGGAT CATGCAGGAA TTTGAAAGTG ACACGTTTTT CCCAGAAATTGATTTGGGGA 2400

AATATAAACT TCTCCCAGAA TACCCAGGCG TCCTCTCTGA GGTCCAGGAGGAAAAAGGCA 2460

TCAAGTATAA GTTTGAAGTC TACGAGAAGA AAGACTAACA GGAAGATGCTTTCAAGTTCT 2520

CTGCTCCCCT CCTAAAGCTA TGCATTTTTT ATAAGACCAT GGGACTTTTGCTGGCTTTAG 2580

ATCATAATCA GCCATACCAC ATTTGTAGAG GTTTTACTTG CTTTAAAAAACCTCCCACAC 2640

CTCCCCCTGA ACCTGAAACA TAAAATGAAT GCAATTGTTG TTGTTAACTTGTTTATTGCA 2700

GCTTATAATG GTTACAAATA AAGCAATAGC ATCACAAATT TCACAAATAAAGCATTTTTT 2760

TCACTGCATT CTAGTTGTGG TTTGTCCAAA CTCATCAATG TATCTTATCATGTCTGGATC 2820

CCCGGCCAAC GGTCTGGTGA CCCGGCTGCG AGAGCTCGGT GTACCTGAGACGCGAGTAAG 2880

CCCTTGAGTC AAAGACGTAG TCGTTGCAAG TCCGCACCAG GTACTGATCATCGATGCTAG 2940

ACCGTGCAAA AGGAGAGCCT GTAAGCGGGC ACTCTTCCGT GGTCTGGTGGATAAATTCGC 3000

AAGGGTATCA TGGCGGACGA CCGGGGTTCG AACCCCGGAT CCGGCCGTCCGCCGTGATCC 3060

ATCCGGTTAC CGCCCGCGTG TCGAACCCAG GTGTGCGACG TCAGACAACGGGGGAGCGCT 3120

CCTTTTGGCT TCCTTCCAGG CGCGGCGGCT GCTGCGCTAG CTTTTTTGGCGAGCTCGAAT 3180

TAATTCTGCA TTAATGAATC GGCCAACGCG CGGGGAGAGG CGGTTTGCGTATTGGGCGCT 3240

CTTCCGCTTC CTCGCTCACT GACTCGCTGC GCTCGGTCGT TCGGCTGCGGCGAGCGGTAT 3300

CAGCTCACTC AAAGGCGGTA ATACGGTTAT CCACAGAATC AGGGGATAACGCAGGAAAGA 3360

ACATGTGAGC AAAAGGCCAG CAAAAGGCCA GGAACCGTAA AAAGGCCGCGTTGCTGGCGT 3420

TTTTCCATAG GCTCCGCCCC CCTGACGAGC ATCACAAAAA TCGACGCTCAAGTCAGAGGT 3480

GGCGAAACCC GACAGGACTA TAAAGATACC AGGCGTTTCC CCCTGGAAGCTCCCTCGTGC 3540

GCTCTCCTGT TCCGACCCTG CCGCTTACCG GATACCTGTC CGCCTTTCTCCCTTCGGGAA 3600

GCGTGGCGCT TTCTCAATGC TCACGCTGTA GGTATCTCAG TTCGGTGTAGGTCGTTCGCT 3660

CCAAGCTGGG CTGTGTGCAC GAACCCCCCG TTCAGCCCGA CCGCTGCGCCTTATCCGGTA 3720

ACTATCGTCT TGAGTCCAAC CCGGTAAGAC ACGACTTATC GCCACTGGCAGCAGCCACTG 3780

GTAACAGGAT TAGCAGAGCG AGGTATGTAG GCGGTGCTAC AGAGTTCTTGAAGTGGTGGC 3840

CTAACTACGG CTACACTAGA AGGACAGTAT TTGGTATCTG CGCTCTGCTGAAGCCAGTTA 3900

CCTTCGGAAA AAGAGTTGGT AGCTCTTGAT CCGGCAAACA AACCACCGCTGGTAGCGGTG 3960

GTTTTTTTGT TTGCAAGCAG CAGATTACGC GCAGAAAAAA AGGATCTCAAGAAGATCCTT 4020

TGATCTTTTC TACGGGGTCT GACGCTCAGT GGAACGAAAA CTCACGTTAAGGGATTTTGG 4080

TCATGAGATT ATCAAAAAGG ATCTTCACCT AGATCCTTTT AAATTAAAAATGAAGTTTTA 4140

AATCAATCTA AAGTATATAT GAGTAAACTT GGTCTGACAG TTACCAATGCTTAATCAGTG 4200

AGGCACCTAT CTCAGCGATC TGTCTATTTC GTTCATCCAT AGTTGCCTGACTCCCCGTCG 4260

TGTAGATAAC TACGATACGG GAGGGCTTAC CATCTGGCCC CAGTGCTGCAATGATACCGC 4320

GAGACCCACG CTCACCGGCT CCAGATTTAT CAGCAATAAA CCAGCCAGCCGGAAGGGCCG 4380

AGCGCAGAAG TGGTCCTGCA ACTTTATCCG CCTCCATCCA GTCTATTAATTGTTGCCGGG 4440

AAGCTAGAGT AAGTAGTTCG CCAGTTAATA GTTTGCGCAA CGTTGTTGCCATTGCTACAG 4500

GCATCGTGGT GTCACGCTCG TCGTTTGGTA TGGCTTCATT CAGCTCCGGTTCCCAACGAT 4560

CAAGGCGAGT TACATGATCC CCCATGTTGT GCAAAAAAGC GGTTAGCTCCTTCGGTCCTC 4620

CGATCGTTGT CAGAAGTAAG TTGGCCGCAG TGTTATCACT CATGGTTATGGCAGCACTGC 4680

ATAATTCTCT TACTGTCATG CCATCCGTAA GATGCTTTTC TGTGACTGGTGAGTACTCAA 4740

CCAAGTCATT CTGAGAATAG TGTATGCGGC GACCGAGTTG CTCTTGCCCGGCGTCAATAC 4800

GGGATAATAC CGCGCCACAT AGCAGAACTT TAAAAGTGCT CATCATTGGAAAACGTTCTT 4860

CGGGGCGAAA ACTCTCAAGG ATCTTACCGC TGTTGAGATC CAGTTCGATGTAACCCACTC 4920

GTGCACCCAA CTGATCTTCA GCATCTTTTA CTTTCACCAG CGTTTCTGGGTGAGCAAAAA 4980

CAGGAAGGCA AAATGCCGCA AAAAAGGGAA TAAGGGCGAC ACGGAAATGTTGAATACTCA 5040

TACTCTTCCT TTTTCAATAT TATTGAAGCA TTTATCAGGG TTATTGTCTCATGAGCGGAT 5100

ACATATTTGA ATGTATTTAG AAAAATAAAC AAATAGGGGT TCCGCGCACATTTCCCCGAA 5160

AAGTGCCACC TGACGTCTAA GAAACCATTA TTATCATGAC ATTAACCTATAAAAATAGGC 5220

GTATCACGAG GCCCTTTCGT CTCGCGCGTT TCGGTGATGA CGGTGAAAACCTCTGACACA 5280

TGCAGCTCCC GGAGACGGTC ACAGCTTGTC TGTAAGCGGA TGCCGGGAGCAGACAAGCCC 5340

GTCAGGGCGC GTCAGCGGGT GTTGGCGGGT GTCGGGGCTG GCTTAACTATGCGGCATCAG 5400

AGCAGATTGT ACTGAGAGTG CACCATATGC GGTGTGAAAT ACCGCACAGATGCGTAAGGA 5460

GAAAATACCG CATCAGGCGC CATTCGCCAT TCAGGCTGCG CAACTGTTGGGAAGGGCGAT 5520

CGGTGCGGGC CTCTTCGCTA TTACGCCAGC TGGCGAAAGG GGGATGTGCTGCAAGGCGAT 5580

TAAGTTGGGT AACGCCAGGG TTTTCCCAGT CACGACGTTG TAAAACGACGGCCAGTGCC 5639